# EUROPEAN PATENT APPLICATION

(11) **EP 4 628 125 A1**
(43) Date of publication of application: **08.10.2025**
(21) Application number: 24168111.3
(22) Date of filing: 02.04.2024
(51) Int. Cl.: A61M 5/315

(54) **SYRINGE PLUNGER STOPPER WITH GUIDANCE-ENABLING INTERNAL CAVITY**

(71) Applicant: Becton, Dickinson and Company, Franklin Lakes, NJ 07417-1880 (US)
(72) Inventor: FLIPPE, Marc, 38640 Claix (FR); VAXELAIRE, Jérémie, 74100 Ambilly (FR)
(74) Representative: Germain Maureau

(57) **Abstract**

Provided herein is a stopper including a main body portion defining an open proximal end and a closed distal end, a plurality of ribs each extending from an outer surface of the main body portion and around an outer circumference of the main body portion, and a cavity formed within the main body, with the cavity extending from the open proximal end distally into the main body. The cavity comprises a threaded cavity portion defined by a threaded inner surface of the main body portion and a rear cavity portion positioned distally from the threaded cavity portion and opposite the open proximal end of the main body portion. The rear cavity portion has a depth of 0.5 mm or greater, such that the rear cavity portion is configured to receive and retain tools therein used for post-manufacturing processing of the stopper and positioning of the stopper within the syringe barrel.

## Description

### BACKGROUND OF THE INVENTION

### Field of the Invention

The present disclosure relates generally to a plunger assembly for use with a syringe and, more particularly, to a plunger stopper constructed to provide maintaining of a consistent alignment and guidance thereof during both manufacturing and vent tube stoppering.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first aspirate the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient. The plunger assembly typically includes an elongated plunger rod extending out of the barrel, and a plunger head or stopper disposed at the distal end of the plunger rod. The stopper is typically made of elastomeric material and is adapted to ensure the container closure integrity (CCI) of a syringe when the stopper is inserted into the syringe.

Existing stopper designs include a stopper body having a tail portion disposed at its proximal end adapted for attachment to the distal end of the plunger rod, and a head portion disposed at its distal end adapted to interfit with the barrel of the syringe. The tail portion of the stopper may include a cavity formed therein configured to receive a distal head of the plunger rod, so as to engage the plunger rod with the stopper. According to some embodiments, each of the cavity and the plunger head may be configured to provide a threaded engagement therebetween to secure the plunger rod to the stopper. The head portion of the stopper may include a plurality of annular, outwardly protruding ribs formed on an external cylindrical wall thereof, with the ribs applying a contact pressure against an inner surface of the syringe barrel to form a seal therewith that ensures the CCI of the syringe. A series of two or three ribs, for example, may be spaced apart longitudinally along the stopper main body, with one or more of the ribs positioned back near the tail portion, such that those rib(s) are formed on the stopper at a location that is axially/longitudinally aligned with where the cavity is formed in the stopper.

In various processes involved with manufacturing the stopper and positioning the stopper within a syringe barrel, it is recognized that maintaining proper alignment or isostatism of the stopper is necessary.

As one example, in post-molding processing of the stopper such as performing a surface treatment on the stopper to form a groove in a rib thereof, it is necessary to maintain a consistent alignment/orientation of the stopper. In existing systems, the stopper may be provided to a surface treatment system by inserting a stabilizing tool or rod into the cavity of the stopper, with the stabilizing rod meant to maintain alignment/orientation of the stopper during treatment.

As another example, in vent tube stoppering of the stopper into a syringe barrel, an installation rod is used to apply a pushing force to the stopper for pushing the stopper out of a vent tube (which is inserted into the syringe barrel) and into the syringe barrel. The installation rod is advanced into the cavity of the stopper, ideally along a center axis of the stopper/cavity, in order to apply a pushing force to the stopper that maintains alignment/orientation of the stopper as it is positioned within the syringe barrel, so as to prevent tilting of the stopper as it is being pushed out of the vent tube and into the syringe barrel.

With existing stopper designs, it is recognized that the cavity formed therein may not be ideally structured to enable maintaining of a consistent alignment/isostatism of the stopper during both manufacturing and vent tube stoppering. That is, the cavity is not structured to adequately accommodate both a stabilizing tool/rod of a post-molding surface treatment system (for example) and an installation rod of a vent tube stoppering system in a manner that ensures maintaining alignment/isostatism of the stopper. Failure to maintain alignment/isostatism of the stopper may lead to manufacturing defects and/or the stopper not forming a proper seal with the syringe barrel when pushed into position.

Accordingly, a need exists in the art for a plunger stopper for use with a syringe, where the stopper is constructed to provide maintaining of a consistent alignment/isostatism of the stopper during both manufacturing (i.e., post-molding treatment) and vent tube stoppering.

### SUMMARY OF THE INVENTION

Provided herein is a stopper adapted for attachment with a plunger rod for use within a syringe barrel. The stopper includes a main body portion defining a proximal end and a distal end, with the proximal end comprising an open end and the distal end comprising a closed end. The stopper also includes a plurality of ribs each extending from an outer surface of the main body portion and around an outer circumference of the main body portion, the plurality of ribs including at least a first rib and a second rib axially spaced from the first rib. A cavity is formed within the main body, the cavity extending from the open proximal end distally into the main body, wherein the cavity comprises a threaded cavity portion defined by a threaded inner surface of the main body portion and a rear cavity portion positioned distally from the threaded cavity portion and opposite the open proximal end of the main body portion. The rear cavity portion has a depth of 0.5 mm or greater, such that the rear cavity portion is configured to receive and retain tools therein used for post-manufacturing processing of the stopper and positioning of the stopper within the syringe barrel.

In certain configurations, a back surface of the rear cavity portion provides an axial contact point for the tool received in the rear cavity portion.

In certain configurations, a side surface of the rear cavity portion provides a first radial contact point for the tool received in the rear cavity portion.

In certain configurations, the threaded inner surface of the main body portion comprises a plurality of thread crests spaced axially along the threaded inner surface, and wherein at least a proximal-most thread crest of the plurality of thread crests provides a second radial contact point for the tool.

In certain configurations, the first radial contact point and the second radial contact point are spaced axially apart from one another by a distance that is 30% or more of an overall depth of the cavity, preferably 50% or more, and more preferably closer to 100%, so as to provide a pair of spaced apart radial contact points between the stopper and the tool.

In certain configurations, the stopper comprises a lubricant-free stopper.

In certain configurations, the stopper comprises one or more layers of inert material applied on the plurality of ribs and the outer surface of the main body portion.

In certain configurations, the inert material formed comprises one or more of a fluoropolymer, an ultra-high-molecular-weight polyethylene (UHMWPE), or a thermoplastic elastomer (TPE) free of per- and poly- fluoroalkyl substances (PFAS).

In certain configurations, each of the rear cavity portion and the threaded cavity portion have a cylindrical shape, and wherein the rear cavity portion has a first diameter that is less than a second diameter of the threaded cavity portion.

In certain configurations, each of the rear cavity portion and the threaded cavity portion have a cylindrical shape, and wherein the rear cavity portion has a diameter that is equal to a diameter of the threaded cavity portion.

Also provided is a method of manufacturing a stopper as previously described. The method includes forming the main body portion and the plurality of ribs via a molding process and performing a post-molding treatment on the stopper. In performing the post-molding treatment, the stopper is placed on an alignment and retention tool, the tool comprising a shaft insertable into the cavity, with the shaft retained and aligned within the cavity via the axial contact point and the first and second radial contact points.

In certain configurations, performing the post-molding treatment comprises performing surface texturing on the stopper.

Also provided is a method of vent tube stoppering a stopper as previously described into a syringe barrel. The method includes providing a vent tube stoppering system including a vent tube configured to be inserted into a chamber of the syringe barrel and having an internal volume and an installation tool moveable inside the internal volume of the vent tube, the installation tool including a distal end. The method also includes positioning the stopper within the internal volume of the vent tube in a compressed state, positioning the vent tube such that a distal portion thereof is positioned within the chamber of the syringe barrel and, with the vent tube in the distal tube operative position, pushing the installation tool distally through the vent tube and into the stopper, so as to push the stopper distally out of the vent tube and into the syringe barrel. In pushing the stopper with the installation tool, the distal end of the installation tool is received in the cavity of the stopper, with the distal end of the installation tool retained and aligned within the cavity via the axial contact point and the first and second radial contact points.

Also provided is a syringe including a syringe barrel having a barrel proximal end and a barrel distal end and defining a chamber configured for containing a fluid therein, and a plunger assembly axially movable within the chamber of the syringe barrel between a retracted position and an advanced position. The plunger assembly further includes a plunger rod having a plunger proximal end and a threaded plunger distal end and a stopper as previously described, wherein the threaded cavity portion is mated with the threaded end of the plunger rod.

In certain configurations, the threaded cavity portion and the threaded end of the plunger rod are configured such that, when the threaded end of the plunger rod is mated with the threaded cavity portion, an interference is present between the threaded end and the threaded cavity portion.

In certain configurations, the plurality of ribs is configured to be in interference is present with an inner surface of the syringe barrel.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of the stopper included in the syringe of FIG. 1, according to a non-limiting embodiment described herein;
FIG. 4 is a side cross-sectional view of the stopper of FIG. 3, taken along line 4-4;
FIG. 5 is a side cross-sectional view of a tool for post-molding treatment of the stopper of FIG. 3, according to a non-limiting embodiment described herein;
FIG. 6 is an exploded perspective view of a vent tube stoppering system for positioning the stopper of FIG. 3 within a syringe barrel, according to a non-limiting embodiment described herein;
FIG. 7 is a front cross-sectional view illustrating a vent tube and an installation rod of the vent tube stoppering system of FIG. 6 in a maximal operative position and a distal rod operative position, respectfully; and
FIG. 8 is a detail view of area I of FIG. 7.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position. Thus, with a syringe for example, the distal end is the end thereof that includes a needle (or luer connection), while the proximal end is where the user engages the plunger (i.e., the plunger thumb press). Similarly, in this application, the terms "in the distal direction" and "distally" mean in the direction toward the distal tip of the syringe, and the terms "in the proximal direction" and "proximally" mean in the direction opposite the direction of the distal tip of the syringe (i.e., in the direction of the plunger thumb press).

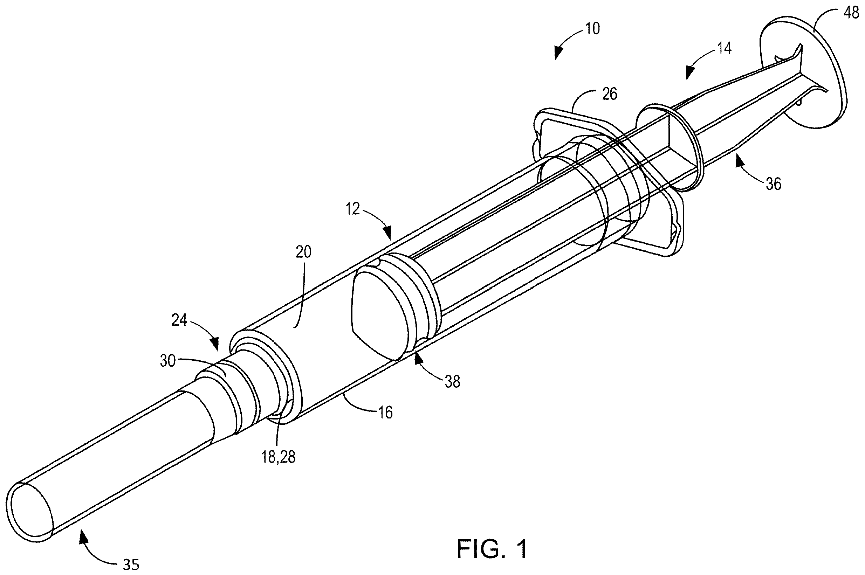

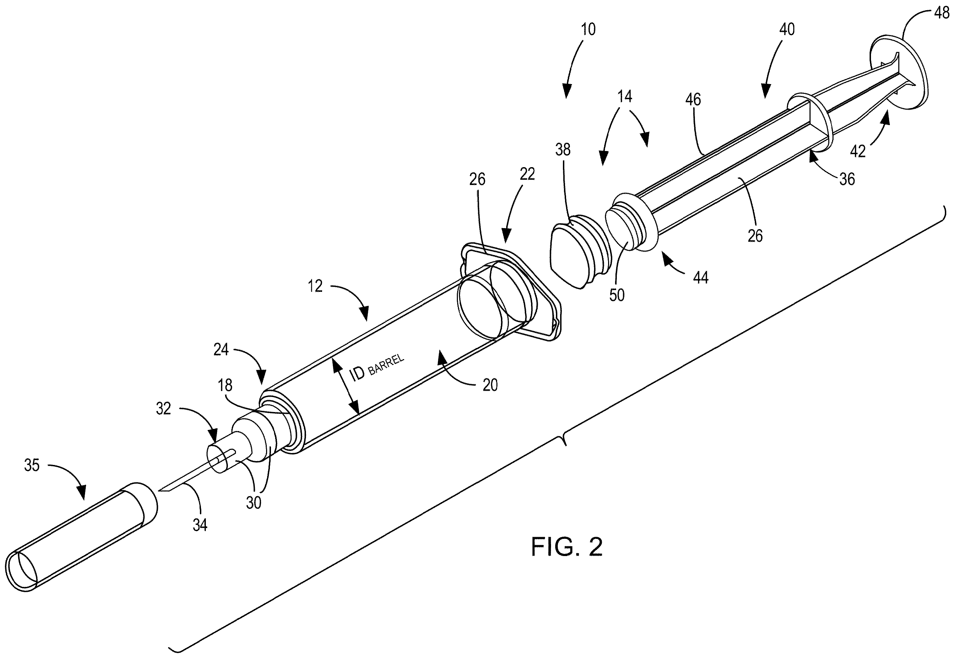

Referring to FIGS. 1 and 2, shown is a non-limiting embodiment of a syringe 10 with which aspects or embodiments of the disclosure may be implemented. According to some aspects of the disclosure, the syringe 10 may be provided as a prefilled syringe, which provides the convenience of rapidly delivering the liquid therein to a patient without the need to first aspirate the medication from another container and meter its volume.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30. According to some aspects of the disclosure, syringe 10 may further include a cover 35 that couples to the hub portion 30 of syringe barrel 12 to protect the needle 34.

The plunger assembly 14 of syringe 10 is formed of an elongate plunger rod 36 and a plunger head or stopper 38. The plunger rod 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger rod 36 with respect to the syringe barrel 12. The plunger distal end 44 includes a threaded extension member 50 that extends out and is configured to mate with the stopper 38. The threaded extension member 50 includes a thread 51 thereon that, according to embodiments, may be configured as a cylindrical thread or a conical thread, as non-limiting examples.

The stopper 38 of plunger assembly 14 is positioned at the plunger distal end 44 so as to be movable along with the plunger rod 36 within the chamber 20 of syringe barrel 12. The stopper 38 may be made from a material that is different from the material of the plunger rod 36 and that is capable of forming a tight seal with the syringe barrel 12 as it is advanced therethrough. In some embodiments, the stopper 38 is formed of an elastomeric material such as butyl, styrene butadiene, isoprene, as non-limiting examples. In other embodiments, the stopper 38 may instead be formed of a polymeric material, such as cross-linked or thermoplastic elastomers, as non-limiting examples. According to aspects of the disclosure, the stopper 38 may be provided as a lubricant-free or low lubricant stopper.

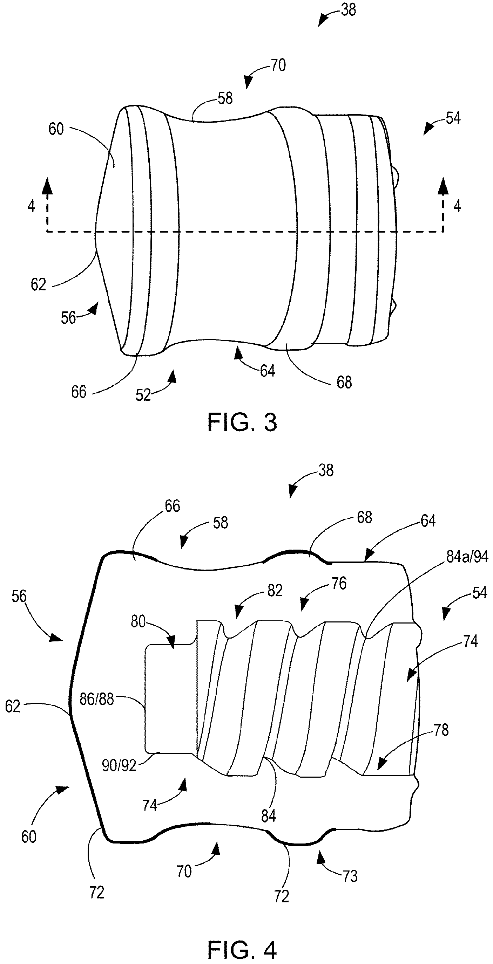

Referring now to FIGS. 3 and 4, and with continued reference to FIGS. 1 and 2, the structure of stopper 38 is shown in greater detail, according to an aspect or embodiment of the disclosure. The stopper 38 is defined by a main body 52 that includes an open proximal end 54 that engages with the distal end of plunger rod 36 and a closed distal end 56 configured to engage with the barrel 12 of syringe 10. The closed distal end 56 of the main body 52 includes a generally cylindrical portion 58 and a roof portion 60 that extends distally from cylindrical portion 58. In one embodiment, the roof portion 60 is configured as a conical portion that extends distally from cylindrical portion 58 to a tip 62 to provide the closed distal end 56. However, it is recognized that roof portion 60 may have other suitable shapes, such as being formed as a flat surface or domed surface, as other non-limiting examples.

An outer surface 64 of the main body 52 includes a plurality of ribs 66, 68 formed thereon that extend circumferentially around the entire outer surface 64. In the illustrated embodiment, the plurality of ribs 66, 68 includes a first rib 66 and a second rib 68, although it is recognized that the stopper 38 could include a greater number of ribs, such as three or four ribs, with it recognized that aspects of the disclosure described here below extend to stoppers with such alternative rib arrangements. The first and second ribs 66, 68 are spaced apart longitudinally, such that the first rib 66 is positioned toward the proximal end 54 of the main body 52 and the second rib 68 is positioned toward the distal end 56 of the main body 52, with an interib region 70 present between the first and second ribs 66, 68.

According to embodiments of the disclosure, the structure of each of first rib 66 and second rib 68 may be controlled to achieve a desired interaction between the stopper 38 and the barrel 12. According to one aspect of ribs 66, 68, the ribs 66, 68 extend radially outward a desired distance from the outer surface 64 (i.e., a rib thickness) to act as bearing points against the inner surface of the syringe barrel 12. In some embodiments, an outer diameter of the ribs, OD_{ribs}, is controlled based on an inner diameter of barrel 12, ID_{barrel} (see FIG. 1), and to provide a desired amount of interference between the stopper 38 and the barrel 12. As one example, the rib thickness and the outer diameter of the ribs, OD_{ribs}, is such that an interference between the stopper 38 and the syringe barrel 12 is in the range of 1.5 to 20%, as determined by: Interference = (OD_{nbs}/ID_{barrel}) - 1) ×100.

According to aspects of the disclosure, the stopper 38 may further include one or more outer coatings or layers 72 of an inert material applied to portions thereof, such as on a sealant or gliding surface thereof (i.e., on a contact surface 73 of first rib 66 and second rib 68) and/or on roof portion 60. The layer(s) 72 may be configured to present an inert surface on those portions of the stopper 38 that may come into contact with the substance contained within syringe barrel 12. In some embodiments, the layer(s) 72 may also be configured to reduce friction between the stopper 38 and barrel 12 when the stopper 38 is moved within the syringe 10. According to non-limiting embodiments, the layer(s) 72 may be formed of a fluoropolymer, an ultra-high-molecular-weight polyethylene (UHMWPE), or a thermoplastic elastomer (TPE) free of per- and polyfluoroalkyl substances (PFAS).

As shown in FIG. 4, the main body 52 of the stopper 38 is substantially hollow and is sized and configured to receive the extension member 50 of plunger rod 36 therein. The main body 52 of the stopper 38 defines an inner cavity 74 that extends from the open proximal end 54 distally into the main body 52. In some embodiments, the inner cavity 74 extends through a majority of a length of the main body 52. The inner cavity 74 may be generally characterized as including a threaded cavity portion 76 (defined by a threaded inner surface 78 of the main body portion 52) and a rear cavity portion 80. In some embodiments, each of the threaded cavity portion 76 and rear cavity portion 80 may be formed as a cylindrical cavity, with identical or different diameters, as described further below.

The threaded cavity portion 76 of cavity 74 is configured to receive and mate with the threaded extension member 50 of plunger rod 36, with the threaded inner surface 78 including a thread 82 thereon that, according to embodiments, may be configured as a cylindrical thread or a conical thread, as non-limiting examples.

The rear cavity portion 80 of cavity 74 is positioned distally from the threaded cavity portion 76 and opposite the open proximal end 54 of the main body portion 52. In some embodiments, the rear cavity portion 80 is configured as a non-threaded portion of the cavity 74. According to some aspects of the disclosure, the rear cavity portion 80 is formed to have a diameter D2 that is the same as the diameter D1 of threaded cavity portion 76. According to other aspects of the disclosure, the rear cavity portion 80 is formed to have a diameter D2 that is the less than the diameter D1 of threaded cavity portion 76.

According to embodiments of the disclosure, the cavity 74 is configured is configured to receive and retain tools therein used for both post-manufacturing treatment of the stopper 38 and positioning of the stopper 38 within the syringe barrel 12 (as part of a vent tube stoppering process). That is, the rear cavity portion 80 and threaded cavity portion 76 of cavity 74 are configured to provide multiple contact points between the stopper 38 and tools inserted therein during treatment and stoppering of the stopper 38. The cavity 74 thus functions as a "dual-purpose" feature for maintaining a consistent alignment and isostatism of the stopper 38 during both manufacturing and vent tube stoppering.

According to aspects of the disclosure, the rear cavity portion 80 of cavity 74 is configured to provide both an axial contact point for a tool and a (first) radial contact point for the tool. The rear cavity portion 80 may be formed to have an axial length or depth of 0.5 mm or greater in order to securely receive a tool therein, while the diameter D2 of the rear cavity portion 80 is formed to be approximately equal to a diameter of the tool. A back surface 86 of the rear cavity portion 80 may thus provide the axial contact point 88 for the tool, while side surfaces 90 of the rear cavity portion 80 provide the (first) radial contact point 92 for the tool.

The threaded cavity portion 76 of cavity 74 is also configured to provide another (or second) contact point for the tool. That is, one or more crests 84 of the thread 82 on the threaded inner surface 78 may be sized/configured to make contact with and provide a (second) radial contact point 94 for the tool. In some embodiments, at least a proximal-most thread crest 84a of the threaded inner surface 78 (adjacent open proximal end 54) provides the (second) radial contact point 94 for the tool. In accordance with one aspect of the disclosure, the first radial contact point 92 and the second radial contact point 94 are spaced axially apart from one another by a distance, A, that is 30% or more of an overall depth of the cavity, B, and preferably 50-60% or more, and even more preferably closer to 100%, so as to provide a pair of spaced apart radial contact points 92, 94 between the stopper 38 and the tool that ensure alignment and isostatism of the stopper 38 on or relative to the tool.

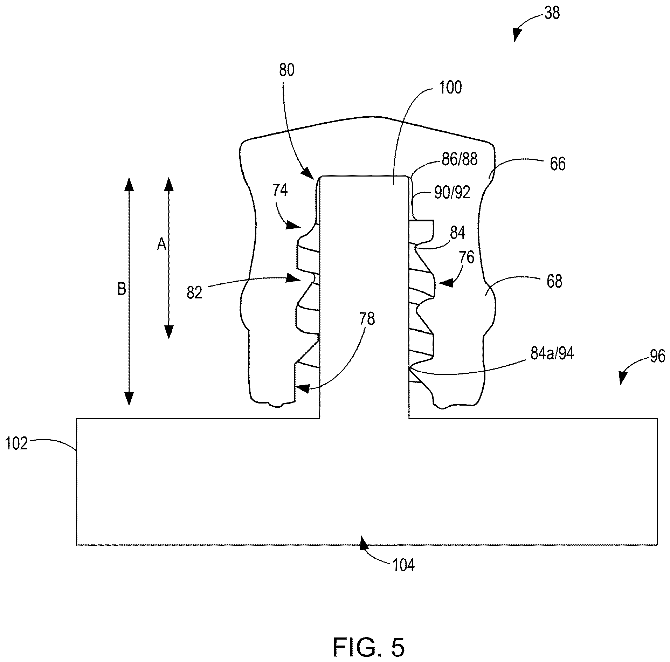

Referring now to FIG. 5, an interaction of stopper 38 and a tool for post-molding processing or treatment of the stopper 38 is shown, in accordance with one aspect of the disclosure. In the illustrated embodiment the tool 96 is provided for performing a post-molding treatment of the surface of the stopper 38, such as a surface texturing on the stopper 38, including to form bumps and/or grooves on the main body 52 or a rib 66, 68 thereof, although it is recognized that the tool 96 could be used for other post-molding treatments. The tool 96 comprises a shaft 100 insertable into the cavity 74 of stopper 38 and a base 102 positioned beneath stopper 38, on which stopper 38 may rest.

As described above, the rear cavity portion 80 and threaded cavity portion 76 of cavity 74 are configured to receive the shaft 100 therein and aid in maintaining a consistent alignment/orientation (isostatism) of the stopper 38 on tool 96. The far end of shaft 100 interfits with the rear cavity portion 80 such that the back surface 86 and side surfaces 90 of the rear cavity portion 80 provide an axial contact point 88 and a (first) radial contact point 92 with the shaft 100. Additionally, one or more of crests 84 of inner threaded surface 78 of the stopper 38 (e.g., a proximal-most crest 84a) provides a (second) radial contact point 94 with the shaft 100. Via the axial contact point 88 and the first and second radial contact points 92, 94 provided between the cavity 74 and the shaft 100, the stopper 38 is maintained in alignment/isostatism on the tool 96 (i.e., on shaft 100) during the post-molding surface treatment thereof.

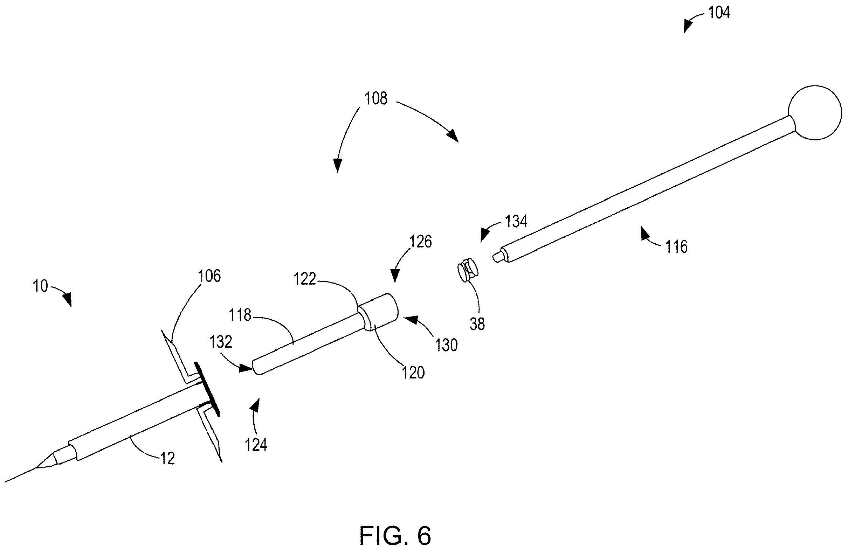

Referring now to FIGS. 6-8, a vent tube stoppering system 104 (hereafter "system 104") is shown that interacts with stopper 38 for positioning the stopper 38 within a syringe barrel 12, in accordance with one aspect of the disclosure . The system 104 may include a container holder 106 configured to receive the proximal end 22 of the syringe barrel 12 to be stoppered (e.g., the flange 26 on the proximal end 22 of syringe barrel 12) and a vent device 108 operable with the container holder 106 to position the stopper 38 into the syringe barrel 12.

The container holder 106 may be a support structure that secures/holds the syringe barrel 12 to allow for insertion of the stopper 38 therein. According to embodiments, the container holder 106 may be provided as a nest that holds a plurality of syringe barrels therein, as known in the art, or may be provided as a holder that is clipped directly onto the stoppering machine.

The vent device 108 includes a vent tube 114 moveable along a longitudinal axis (A) and an installation tool or rod 116 moveable inside the vent tube 114 along the longitudinal axis (A), for positioning a stopper 38 into the syringe barrel 12 to be stoppered. The vent tube 114 is of an elongated shape, preferably of a substantially circular section, and comprises two sections 118, 120 (i.e., a first section 118 and a second section 120) separated by a shoulder 122, with an outer diameter of the second section 120 of the vent tube 114 being greater than the outer diameter of the first section 118. The first section 118 is provided at a distal end 124 of the vent tube 114 and is configured to have a smaller diameter than the syringe barrel 12, such that the first section 118 can be inserted into the syringe barrel 12. The second section 120 is provided at a proximal end 126 of the vent tube 114 and is configured to have a larger diameter than the syringe barrel 12, such that the second section 120 is prevented from being inserted into the syringe barrel 12, but instead serves as a stop.

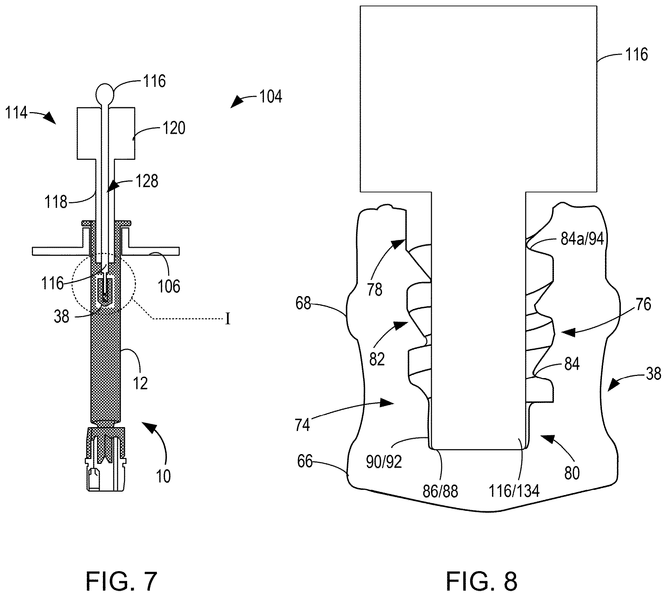

As shown in FIG. 7, the vent tube 114 defines an internal passageway 128 therein that extends through a length of the vent tube 114 and that is configured to receive a compressed stopper 38 therein. Upon placement of the stopper 38 inside the vent tube 114, the first section 118 of the vent tube 114 may be inserted into the syringe barrel 12 (which is secured by container holder 106). In more detail, the vent tube 114 is movable between a proximal tube rest position where the distal end 124 of the vent tube 114 is located outside the syringe barrel 12, and a distal tube operative position where the distal end 124 of the vent tube 114 enters into the syringe barrel 12 via the open proximal end 22 thereof, so as to be relatively close to the surface of a composition contained in the syringe barrel 12.

When the vent tube 114 is advanced in the distal direction to the distal tube operative position, the first section 118 of the vent tube 114 slides inside the syringe barrel 12 along the axis (A) until the shoulder 122 between the first and the second sections 118, 120 of the vent tube 114 abuts the proximal end 22 of the syringe barrel 12. The vent tube 114 then cannot slide further towards the syringe barrel 12. This position of the vent tube 114 relative to the syringe barrel 12 corresponds to a maximal operative position wherein the distal end 124 of the vent tube 114 is located inside the syringe barrel 12 at a determined position.

In this maximal operative position, the installation rod 116 is then pushed in the distal direction and through vent tube 114, thereby pushing the stopper 38 out of the vent tube 114 through the distal opening 132 of the vent tube 114. In use of the installation rod 116, the installation rod 116 is inserted into the vent tube 114 via proximal opening 130. As the outer diameter of the installation rod 116 is smaller than the inner diameter of the internal passageway 128 the installation rod 116 may slide inside the vent tube 114 and move along the axis (A) relative to the vent tube 114, both in a proximal and in a distal direction. To allow the positioning of the stopper 38 in the syringe barrel 12, the length of the installation rod 116 is longer than the length of the vent tube 114 and is adapted so that a distal end 134 of the installation rod 116 sufficiently protrudes from the distal end 124 of the vent tube 114 for pushing the stopper 38 in a desired position into the syringe barrel 12.

After the vent tube 114 (with the stopper 38 therein) is advanced in the distal direction up to the maximal operative position, the installation rod 116 is pushed also in the distal direction, from a proximal rod rest position to a distal rod operative position, wherein the installation rod 116 pushes the stopper 38 out of the vent tube 114 via the distal opening 132 thereof. That is, the distal end 134 of the installation rod 116 is configured to engage the stopper 38, so that the installation rod 116 is able to effectively apply a pushing force to the stopper 38 (to push the stopper out from vent tube 114 and into syringe barrel 12). As a result, the stopper 38 is mechanically positioned in the syringe barrel 12 at a determined distance from the surface of the composition contained herein, and passes from a contracted state wherein stopper 38 is pressed radially against the inner surface of the vent tube 114 (in passageway 128) and has a reduced diameter, to a loosened state wherein stopper 38 expands radially and contacts the inner surface of the syringe barrel 12.

As described above, and as shown best in FIG. 8, the rear cavity portion 80 and threaded cavity portion 76 of cavity 74 are configured to receive the distal end 134 of the installation rod 116 therein and aid in maintaining a consistent alignment/orientation (isostatism) of the stopper 38 on the installation rod 116 as the stopper 38 is pushed out of the vent tube 114 and into syringe barrel 12. The distal-most end of the installation rod 116 interfits with the rear cavity portion 80, such that the back surface 86 and side surfaces 90 of the rear cavity portion 80 provide an axial contact point 88 and a (first) radial contact point 92 with the installation rod 116. Additionally, one or more of crests 84 of the inner threaded surface 78 of the stopper 38 (e.g., a proximal-most crest 84a) provides a (second) radial contact point 94 with the installation rod 116. Via the axial contact point 88 and the first and second radial contact points 92, 94 provided between the cavity 74 and the installation rod 116, the stopper 38 is maintained in alignment/isostatism on the installation rod 116 as the installation rod 116 pushes the stopper 38 out of vent tube 114 and into syringe barrel 12.

In addition to cavity 74 functioning to provide/maintain a consistent alignment and isostatism of the stopper 38 during both manufacturing and vent tube stoppering, it is recognized that the structure of the cavity 74 also reduces a level of the pushing/insertion force required to insert the stopper 28 into the syringe barrel 12 during vent tube stoppering. In some embodiments, the insertion force may be reduced by 40-45% (e.g., from 23 N to 13 N) during vent tube stoppering, as a non-limiting example.

Beneficially, embodiments of the invention thus are directed to a stopper adapted for attachment with a plunger rod for use within a syringe barrel, with the stopper including a guidance-enabling cavity therein that aids in maintaining isostatism of the stopper. The cavity of the stopper functions as a "dual-purpose" feature for maintaining a consistent alignment and isostatism of the stopper during both during post-molding treatment and vent tube stoppering of the stopper. The cavity includes a threaded cavity portion and a rear cavity portion that provide multiple contact points between the cavity and a tool inserted in the cavity for facilitating treatment or stoppering. An axial contact point and multiple radial contact points are provided between the cavity and the tool that maintain the stopper in alignment/isostatism with the tool.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A stopper adapted for attachment with a plunger rod for use within a syringe barrel, the stopper comprising:
a main body portion defining a proximal end and a distal end, the proximal end comprising an open end and the distal end comprising a closed end; and
a plurality of ribs each extending from an outer surface of the main body portion and around an outer circumference of the main body portion, the plurality of ribs including at least a first rib and a second rib axially spaced from the first rib;
a cavity formed within the main body, the cavity extending from the open proximal end distally into the main body, wherein the cavity comprises:
a threaded cavity portion defined by a threaded inner surface of the main body portion; and
a rear cavity portion positioned distally from the threaded cavity portion and opposite the open proximal end of the main body portion;
wherein the rear cavity portion has a depth of 0.5 mm or greater, such that the rear cavity portion is configured to receive and retain tools therein used for post-manufacturing processing of the stopper and positioning of the stopper within the syringe barrel.

2. The stopper of claim 1, wherein a back surface of the rear cavity portion provides an axial contact point for the tool received in the rear cavity portion.

3. The stopper of claim 1 or claim 2, wherein a side surface of the rear cavity portion provides a first radial contact point for the tool received in the rear cavity portion.

4. The stopper of any of claims 1-3, wherein the threaded inner surface of the main body portion comprises a plurality of thread crests spaced axially along the threaded inner surface, and wherein at least a proximal-most thread crest of the plurality of thread crests provides a second radial contact point for the tool.

5. The stopper of claim 4, wherein the first radial contact point and the second radial contact point are spaced axially apart from one another by a distance of by a distance that is 30% or more of an overall depth of the cavity, preferably 50% or more, and more preferably closer to 100%, so as to provide a pair of spaced apart radial contact points between the stopper and the tool.

6. The stopper of any of claims 1-6, wherein the stopper comprises a lubricant-free stopper.

7. The stopper of any of claims 1-6, wherein the stopper comprises one or more layers of inert material applied on the plurality of ribs and the outer surface of the main body portion.

8. The stopper of claim 7, wherein the inert material formed comprises one or more of a fluoropolymer, an ultra-high-molecular-weight polyethylene (UHMWPE), or a thermoplastic elastomer (TPE) free of per- and poly- fluoroalkyl substances (PFAS).

9. The stopper of any of claims 1-8, wherein each of the rear cavity portion and the threaded cavity portion have a cylindrical shape, and wherein the rear cavity portion has a first diameter that is less than a second diameter of the threaded cavity portion.

10. The stopper of any of claims 1-8, wherein each of the rear cavity portion and the threaded cavity portion have a cylindrical shape, and wherein the rear cavity portion has a diameter that is equal to a diameter of the threaded cavity portion.

11. A method of manufacturing the stopper of any of claims 1-10, the method comprising:
forming the main body portion and the plurality of ribs via a molding process; and
performing a post-molding treatment on the stopper;
wherein, in performing the post-molding treatment, the stopper is placed on an alignment and retention tool, the tool comprising a shaft insertable into the cavity, with the shaft retained and aligned within the cavity via the axial contact point and the first and second radial contact points.

12. The method of claim 11, wherein performing the post-molding treatment comprises performing a surface texturing on the stopper.

13. A method of vent tube stoppering the stopper of any of claims 1-10 into a syringe barrel, the method comprising:
providing a vent tube stoppering system comprising:
a vent tube configured to be inserted into a chamber of the syringe barrel, the vent tube having an internal volume; and
an installation tool moveable inside the internal volume of the vent tube, the installation tool including a distal end;
positioning the stopper within the internal volume of the vent tube in a compressed state;
positioning the vent tube such that a distal portion thereof is positioned within the chamber of the syringe barrel; and
with the vent tube in the distal tube operative position, pushing the installation tool distally through the vent tube and into the stopper, so as to push the stopper distally out of the vent tube and into the syringe barrel;
wherein, in pushing the stopper with the installation tool, the distal end of the installation tool is received in the cavity of the stopper, with the distal end of the installation tool retained and aligned within the cavity via the axial contact point and the first and second radial contact points.

14. A syringe comprising:
a syringe barrel having a barrel proximal end and a barrel distal end and defining a chamber configured for containing a fluid therein; and
a plunger assembly axially movable within the chamber of the syringe barrel between a retracted position and an advanced position, the plunger assembly including:
a plunger rod having a plunger proximal end and a plunger distal end, the plunger rod distal end comprising a threaded end; and
the stopper of claim 1, wherein the threaded cavity portion is mated with the threaded end of the plunger rod.

15. The syringe of claim 14, wherein the threaded cavity portion and the threaded end of the plunger rod are configured such that, when the threaded end of the plunger rod is mated with the threaded cavity portion, an interference is present between the threaded end and the threaded cavity portion.

16. The syringe of any of claims 1-9, wherein the plurality of ribs is configured to be in interference is present with an inner surface of the syringe barrel.
